# EUROPÄISCHE PATENTSCHRIFT

(11) **EP 2 228 027 B1**
(45) Veröffentlichungstag und Bekanntmachung des Hinweises auf die Patenterteilung: **28.03.2012**
(21) Anmeldenummer: 10150291.2
(22) Anmeldetag: 08.01.2010
(51) Int. Cl.: A61B 18/14, H01R 13/627

(54) **Hochfrequenz-Chirurgiegerät**
High frequency surgical device
Appareil chirurgical haute fréquence

(30) Priorität: 10.03.2009 DE 102009011912
(43) Veröffentlichungstag der Anmeldung: 15.09.2010
(73) Patentinhaber: Celon AG Medical Instruments, 14513 Teltow (DE)
(72) Erfinder: Kühne, Wolfgang, 16567, Schönfließ (DE)
(74) Vertreter: von Oppen, Joachim F.M.

(56) Entgegenhaltungen:
- DE-A1- 3 209 474
- GB-A- 2 292 264
- US-A- 4 936 842
- US-A1- 2004 097 117
- US-A1- 2007 032 789

## Beschreibung

Die Erfindung betrifft ein Hochfrequenz-Chirurgiegerät zum Erzeugen von Hochfrequenz-Energie für das Trennen und/oder Koagulieren von biologischem Gewebe, mit wenigstens einer Energiequelle und mit wenigstens einer Ausgangsbuchse, in die ein langgestreckter elektrisch leitender Steckkontakt eines elektrochirurgischen Instruments einsteckbar ist und die eine Raste zum Einrasten in eine im Steckkontakt ausgebildete Gegenraste umfasst.

Hochfrequenz- oder HF-Chirurgiegeräte dieser Art sind seit langem aus dem Stand der Technik bekannt und werden auch als HF Generator bezeichnet. Zum Trennen bzw. Schneiden und/oder Koagulieren von biologischem Gewebe erzeugt das HF-Chirurgiegerät eine HF Ausgangsenergie. Verschiedenste monopolare oder bipolare Instrumente können an das HF-Chirurgiegerät angeschlossen werden, welche die HF Ausgangsenergie in das biologische Gewebe eines zu behandelnden Patienten einleiten. Im oder am Gewebe bewirkt die HF Energie das gewünschte elektrochirurgische Trennen oder Koagulieren.

Aus US 4 936 842 A ist ein Hochfrequenz-Chirurgiegerät zum Erzeugen von Hochfrequenz-Energie für das Trennen und/oder Koagulieren von biologischem Gewebe bekannt, das eine Energiequelle und mit wenigstens einer Ausgangsbuchse besitzt, in die ein proximales Ende eines elektrochirurgischen Instruments eingesteckt werden kann und die eine Raste zum Einrasten in eine im Steckkontakt ausgebildete Gegenraste aufweisst, sowie einen von der Raste elektrisch isolierten Buchsenkontakt, der mit der Energiequelle elektrisch verbunden ist.

Zum Übertragen der erzeugten HF Energie an das elektrochirurgische Instrument weist das HF Chirurgiegerät, häufig an der Frontseite, eine oder mehrere Ausgangsbuchsen auf. In die Ausgangsbuchsen können standardisierte Steckkontakte, wie beispielsweise der sogenannte Bovie Stecker oder der Olympus 6mm Rundstecker, die Teil des elektrochirurgischen Instruments sind, eingesteckt werden. Im eingesteckten Zustand ist der elektrisch leitende Steckkontakt mit der Energiequelle im Innern des HF Chirurgiegeräts elektrisch verbunden. So kann das elektrochirurgische Instrument im Betrieb mit HF Energie beaufschlagt werden.

Um den Steckkontakt in der Ausgangsbuchse des HF Chirurgiegeräts zu fixieren, greift eine Raste in Gegenraste des Steckkontakts ein, wenn der Steckkontakt in die Ausgangsbuchse eingeführt wird. Gleichzeitig stellt die Raste bei Geräten im Stand der Technik den elektrischen Kontakt zwischen dem HF Chirurgiegerät und dem metallischen Steckkontakt her.

Die HF Chirurgiegeräte nach dem beschriebenen Stand der Technik haben den Nachteil, dass es zum unerwünschten Kontakt eines Benutzers mit einem nur zum Teil eingesteckten Steckkontakt kommen kann, wenn der Steckkontakt bereits mit der Energiequelle des HF Chirurgiegeräts verbunden ist. Bei einem solchen unerwünschten Kontakt ist eine Gefährdung des Benutzers nicht auszuschließen.

Daher ist es die Aufgabe der vorliegenden Erfindung, ein Hochfrequenz-Chirurgiegerät bereitzustellen, das die Sicherheit des Benutzers erhöht.

Diese Aufgabe ist bei dem erfindungsgemäßen Hochfrequenz-Chirurgiegerät nach Anspruch 1 durch einen von der Raste elektrisch isolierten Buchsenkontakt gelöst, der mit der Energiequelle elektrisch verbunden ist.

Die erfindungsgemäße Lösung hat den Vorteil, dass durch die elektrisch von der Energiequelle isolierte Raste keine Energie an den Steckkontakt übertragen werden kann. Die elektrische Verbindung zur Energiequelle wird erst durch den Kontakt mit dem separaten Buchsenkontakt hergestellt. Der Buchsenkontakt ist so angeordnet, dass beim Einschieben der elektrische Kontakt zum Steckkontakt erst hergestellt wird, wenn die Einstecktiefe des Steckkontakts einen Kontakt des Benutzers ausschließt.

Der größtmögliche Abstand von einem Kontaktpunkt mit dem Steckkontakt zur Außenseite des Hochfrequenz-Chirurgiegeräts ist beim Buchsenkontakt größer als bei der Raste. Dies hat den Vorteil, dass beim Einführen des Steckkontakts in die Ausgangsbuchse der elektrische Kontakt genügend spät erfolgen kann und trotzdem die Raste früh eingreifen kann, um die Fixierung zu garantieren. Die Erfindung kann durch verschiedene vorteilhafte Weiterbildungen ergänzt werden, die im Folgenden beschrieben sind.

Um diese möglichst späte elektrische Kontaktierung auch für Steckkontakte mit kugel- oder halbkugelförmigen axialen Enden zu realisieren, kann der Buchsenkontakt so angeordnet sein, dass er den Steckkontakt in eingestecktem Zustand am axialen Ende des Steckkontakts kontaktiert.

Weiterhin kann der Buchsenkontakt so angeordnet sein, dass er den Steckkontakt beim Einschieben erst kontaktiert nachdem die Raste im Wesentlichen vollständig in die Gegenraste eingerastet ist. Dies hat den Vorteil, dass der Steckkontakt in die Kontaktposition gezogen wird. So kann ein Fehler durch einen gerasteten Steckkontakt ohne elektrischen Kontakt ausgeschlossen werden.

Um einen sicheren elektrischen Kontakt zwischen Buchsenkontakt und Steckkontakt herzustellen, kann der Buchsenkontakt aus einem federnden Material hergestellt und vom Steckkontakt im eingesteckten Zustand federnd auslenkbar angeordnet sein. Die resultierende Federkraft im eingesteckten Zustand erhöht den elektrischen Fluss. Darüber hinaus kann die Raste aus einem federnden Material hergestellt sein, wobei die Federkonstante der Raste größer ist als die des Buchsenkontakts. Dies hat den Vorteil, dass der Buchsenkontakt den Steckkontakt nicht entgegen der Einsteckrichtung aus der Verrastung herausdrücken kann.

Es ist von Vorteil, die Kontaktfläche mit einem an der axialen Spitze kugelförmigen oder balligen Steckkontakt eines einpoligen Rundsteckers zu vergrößern. Solche Rundstecker sind beispielsweise der sogenannte Bovie Stecker oder der Olympus 6mm Rundstecker. Hierfür kann der Buchsenkontakt im Wesentlichen gabelförmig oder geschlitzt ausgestaltet sein und so angeordnet sein, dass die Achse des rotationssymmetrischen Steckkontakts vom Kontakt ausgespart ist. Hierdurch können nämlich zwei Kontaktbereiche realisiert werden, welche die elektrische Kontaktfläche vergrößern.

Im Folgenden wird die Erfindung anhand der in den Zeichnungen dargestellten beispielhaften Ausführungsformen erläutert. Die unterschiedlichen Merkmale können, wie auch bei den oben beschriebenen Ausführungsformen, beliebig miteinander kombiniert werden.

Es zeigen:
- Fig. 1: eine schematische Darstellung einer beispielhaften Ausführungsform eines erfindungsgemäßen Hochfrequenz-Chirurgiegerätes;
- Fig.2: eine schematische Schnittdarstellung einer Ausgangsbuchse des Hoch- frequenz-Chirurgiegerätes nach Fig. 1 mit einem eingeführten Steckkontakt in einer ersten Position;
- Fig. 3: eine schematische Schnittdarstellung der Ausgangsbuchse des Hoch- frequenz-Chirurgiegerätes nach Fig. 1 mit dem eingeführten Steckkontakt in einer zweiten Position;
- Fig.4: eine schematische Darstellung eines Buchsenkontakts des Hochfrequenz- Chirurgiegerätes nach Fig. 1 im Schnitt A-A;
- Fig. 5: eine schematische Schnittdarstellung der Ausgangsbuchse des Hoch- frequenz-Chirurgiegerätes nach Fig. 1 mit dem eingeführten Steckkontakt in einer dritten Position.

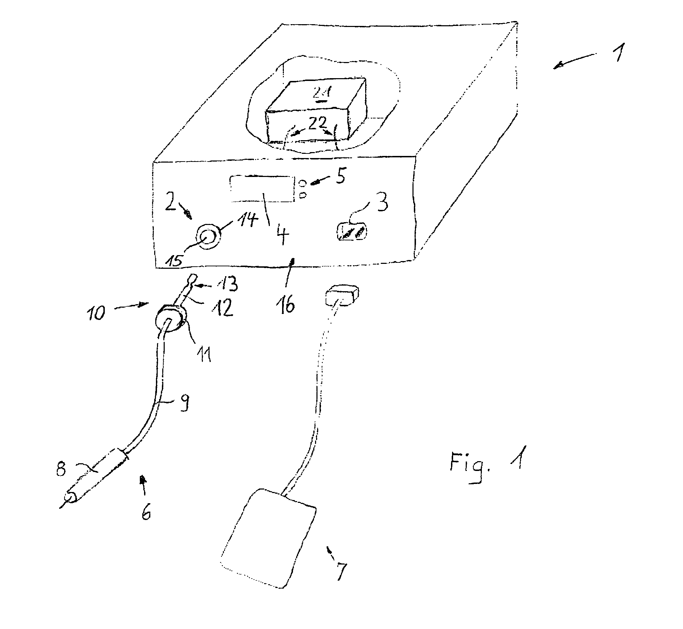

Zunächst wird der allgemeine Aufbau eines erfindungsgemäßen HF Chirurgiegerätes 1 anhand der Ausführungsform in Fig. 1 dargestellt.

Das HF Chirurgiegerät 1 umfasst, jeweils an der Außenseite 25 angeordnet, eine Ausgangsbuchse 2, eine Eingangsbuchse 3, eine Visualisierungsanzeige 4 und Bedienelemente 5.

Im Betrieb erzeugt das HF Chirurgiegerät 1 eine Hochfrequenz-Energie für das Trennen und/oder Koagulieren von biologischen Geweben. Dabei wird eine hochfrequente Wechselspannung zwischen der Ausgangsbuchse 2 und der Eingangsbuchse 3 bereitgestellt. Im Innern umfasst das HF Chirurgiegerät 1 eine Energiequelle 21, die mit der Eingangsbuchse 3 und der Ausgangsbuchse 2 jeweils über ein Verbindungskabel 22 verbunden ist. Die Energiequelle 21 ist in Fig. 1 lediglich sehr stark vereinfacht dargestellt. Die Eingangsbuchse 3 ist, wie im Stand der Technik üblich, als eine standardisierte Buchse für die Verbindung mit einem Anschlussstecker einer Rückleitelektrode ausgebildet. Die Ausgangsbuchse 2 wir im Folgenden noch detailliert beschrieben. Die Hochfrequenz-energie kann an den Bedienelementen 5 eingestellt werden. So kann beispielsweise die Leistung oder der Betriebsmodus variiert werden. Eine visuelle Anzeige einiger Einstellungen erhält der Bediener über die Visualisierungsanzeige 4.

Für das Trennen und/oder Koagulieren von biologischen Geweben kann an die Ausgangsbuchse 2 ein geeignetes elektrochirurgisches Instrument 6 und an die Eingangsbuchse 3 eine geeignete Rückleitelektrode 7 angeschlossen werden. In Fig. 1 ist das elektrochirurgische Instrument 6 beispielsweise ein elektrisches Skalpell zum Schneiden von Gewebe. Selbstverständlich kann auch jedes andere elektrochirurgische Instrument angeschlossen werden.

Das Instrument 6 weist ein Handstück 8, ein Kabel 9 und einen Anschlussstecker 10 auf. Der Anschlussstecker 10, der in Fig. 1 als einpoliger Rundstecker, insbesondere dem sogenannten Bovie Stecker, ausgebildet ist, weist einen Gehäusekörper 11 und einen metallischen, elektrisch leitenden Steckkontakt 12 auf. Am distalen Ende des im Wesentlichen zylindrisch ausgebildeten Steckkontakts 12 ist eine Gegenraste 13 vorgesehen. Die Gegenraste 13 ist als ein im Wesentlichen konkaver Einstich in Umfangsrichtung des rotationssymmetrischen Steckkontakts 12 ausgebildet. Der äußere Durchmesser des Steckkontakts 12 ist bei der Ausführungsform in Fig. 1 etwa 8 mm. Das distale Ende des Steckkontakts 12 ist im Wesentlichen kugelförmig ausgeformt.

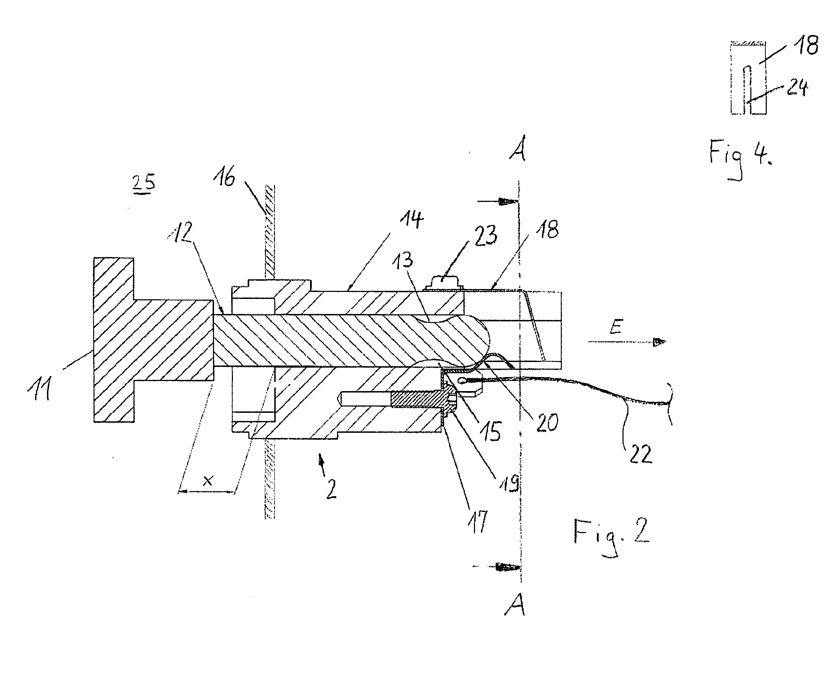

Die Ausgangsbuchse 2 weist einen elektrisch nicht leitenden Buchsenkörper 14, der bei der Ausführungsform in Fig. 1 bis 5 aus Kunststoff hergestellt ist. Der Buchsenkörper 14 weist einen im Wesentlichen zylindrischen Führungskanal 15 auf, in dem der Steckkontakt 12 aufgenommen werden kann. Im Folgenden wir der strukturelle Aufbau der Ausgangsbuchse 2 insbesondere auch anhand der Fig. 2 bis 5 beschrieben.

Fig. 2, 3 und 5 zeigen die an der Frontplatte 16 des HF Chirurgiegerätes 1 befestigte Ausgangsbuchse 2 im Schnitt. Die Ausgangsbuchse 2 weist neben dem Buchsenkörper 14 mit dem Führungskanal 15, eine Raste 17 und einen Buchsenkontakt 18 auf.

Die Raste 17 ist aus einem streifenförmigen Federstahl hergestellt und an einem Ende am Buchsenkörper 14 mittels einer Schraube 19 fixiert. An dem anderen Ende ist der Federstahl zu einer Rastnase 20 gebogen. Die Rastnase 20 ragt im nicht ausgelenkten Zustand, wie in Fig. 2 dargestellt, in den Führungskanal 15 bzw. in die Projektion des Führungskanals 15 in einer Einsteckrichtung E hinein. Im Bereich der Rastnase 20 ist der Führungskanal 15 im Buchsenkörper 14 geöffnet, damit die Rastnase 20 in die Gegenraste 13 des Steckkontakts 12 eingreifen kann.

Der Buchsenkontakt 18 ist aus einem streifenförmigen, elektrisch gut leitenden Metallblech, wie beispielsweise Kupfer oder Bronze, hergestellt und mit einer Schraube 23 am Buchsenkörper 14 befestigt. An einem Ende weist der Buchsenkontakt 18 eine Kontaktlasche 24 auf, an welcher der Buchsenkontakt 18 über das Verbindungskabel 22 mit der Energiequelle 21 elektrisch verbunden ist. Am anderen Ende ist der Buchsenkontakt 22 gekröpft ausgeformt und ragt in den Führungskanal 15 hinein. Wie in Fig. 4 dargestellt, weist der Buchsenkontakt 18 an diesem Ende einen geraden Schlitz 24 auf, der den Buchsenkontakt 18 teilt und dadurch gabelförmig ausbildet. Ähnlich wie bei der Raste 17 ist der Führungskanal 15 im Bereich des Buchsenkontakts 18 geöffnet, damit der gekröpfte Buchsenkontakt 18 in den Führungskanal 15 hineinragen kann.

In Fig. 2 ist die Ausgangsbuchse 2 mit dem eingeführten Steckkontakt 12 in einer Erstkontaktposition mit der Raste 17 dargestellt. Um in diese Position zu gelangen, wird der Anschlussstecker 10 in der Einsteckrichtung E in den Führungskanal 15 eingeführt bis er die Raste 17 kontaktiert, aber noch nicht auslenkt. Dabei befindet sich ein Abschnitt des Steckkontakts 12 mit der Länge x außerhalb des Führungskanals 15. Bei Geräten aus dem Stand der Technik, wird in dieser Position über die Raste die elektrische Verbindung zur Energiequelle 21 hergestellt. Dabei ist die Länge x so groß, dass ein Benutzer den elektrisch bereits mit der Energiequelle verbundenen Steckkontakt 12 beispielsweise mit seinem Finger berühren kann. Bei der Ausführungsform in Fig. 2 ist die kritische Länge x beispielsweise etwa 9 mm. Bei solchen HF Chirurgiegeräten aus dem Stand der Technik ist kein ausreichender Berührschutz gegeben. Diese Gefahr ist bei dem erfindungsgemäßen HF Chirurgiegerät 1 ausgeschlossen, weil die Raste 17 elektrisch von der Energiequelle 21 getrennt ist. In der in Fig. 2 dargestellten Position ist der Steckkontakt 12 noch nicht mit der Energiequelle 21 verbunden.

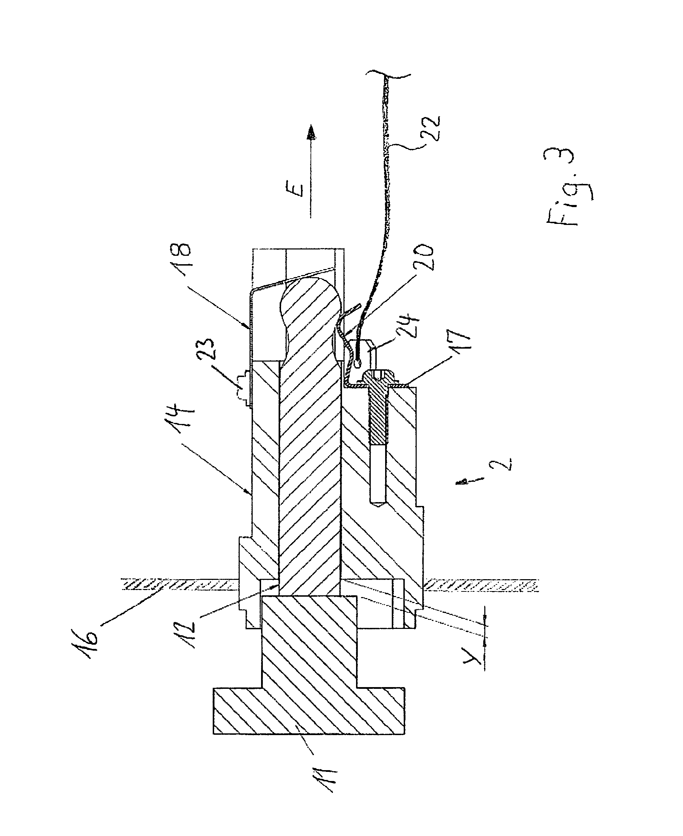

Bei dem erfindungsgemäßen HF Chirurgiegerät 1 wird die elektrische Verbindung zur Energiequelle 21 nämlich erst in der in Fig. 3 dargestellten Erstkontaktposition mit dem Buchsenkontakt 18 hergestellt. Im Vergleich zu der Position in Fig. 2 ist der Steckkontakt 12 in Fig. 3 weiter in den Führungskanal 15 hineingeschoben. In Fig. 3 steht der Steckkontakt 12 nur noch mit einer Länge y aus den Führungskanal 15 nach außen heraus. Wobei y kleiner ist als x und zu klein ist, dass der Finger eines Benutzers den metallischen Steckkontakt 12 berühren kann. Zur Prüfung kann ein sogenannter Normfinger verwendet werden, der einen durchschnittlichen Finger eines Benutzers verkörpert. Bei der Ausführungsform in Fig. 3 ist y beispielsweise etwa 2 mm. In der Position in Fig. 3 greift die Rastnase 20 bereits vollständig in die Gegenraste 13 ein, so dass der Anschlussstecker 10 nicht mehr aus der Ausgangsbuchse 2 herausrutschen kann. Im Gegenteil, sobald die Rastnase 20 in die Gegenraste 13 eingreift, erzeugt die Federkraft der Raste 17 eine Kraftkomponente in der Einsteckrichtung E, welche den Steckkontakt 12 gegen den Buchsenkontakt 18 drückt.

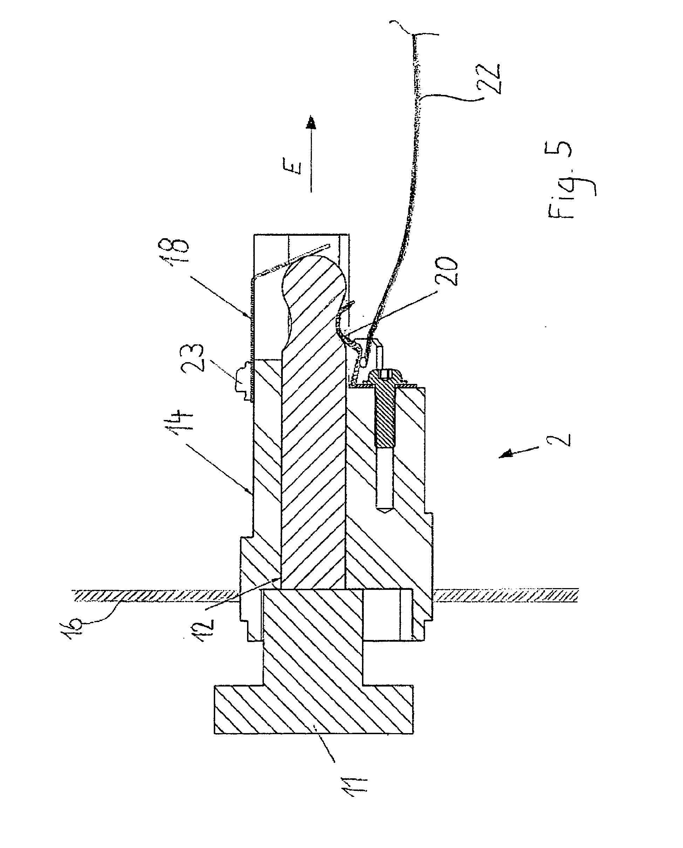

In Fig. 5 zeigt die Ausgangsbuchse 2 mit dem eingeführten Steckkontakt 12 in einer Endposition, in der die Raste 17 im Wesentlichen vollständig in die Gegenraste 13 eingreift. Der eingerastete Anschlussstecker 10 ist in dieser Position im HF Chirurgiegerät 1 fixiert. Der Steckkontakt 13 lenkt den Buchsenkontakt 18 in der Einsteckrichtung E federnd aus, so dass der Buchsenkontakt 18 gegen das kugelförmige Ende des Steckkontakts 13 drückt. So ist ein guter elektrischer Kontakt zwischen Buchsenkontakt 18 und Steckkontakt 13 gewährleistet. Um ein Zurückschieben des Steckkontakts 18 aus der Rastposition in Fig. 3 heraus zu verhindern, ist die Federkonstante k₁ der Raste 17 größer als die Federkonstante k₂ des Buchsenkontakts 18. Daher ist die Kraftkomponente entgegen der Einsteckrichtung E durch den Buchsenkontakt 18 kleiner als die Kraftkomponente in der Einsteckrichtung E durch die Raste 17.

## Patentansprüche

1. Hochfrequenz-Chirurgiegerät (1) zum Erzeugen von Hochfrequenz-Energie für das Trennen und/oder Koagulieren von biologischem Gewebe, mit wenigstens einer Energiequelle (21) und mit wenigstens einer Ausgangsbuchse (2), in die ein langgestreckter elektrisch leitender Steckkontakt (12) eines elektrochirurgischen Instruments (6) einsteckbar ist und die eine Raste (17) zum Einrasten in eine im Steckkontakt ausgebildete Gegenraste (13) umfasst, und einem von der Raste (17) elektrisch isolierten Buchsenkontakt (3), der mit der Energiequelle (21) elektrisch verbunden ist,
**dadurch gekennzeichnet, dass**
der größtmögliche Abstand von einem Kontaktpunkt mit dem Steckkontakt (12) zur Außenseite des Hochfrequenz-Chirurgiegeräts (1) beim Buchsenkontakt (3) größer ist als bei der Raste(17).

2. Hochfrequenz-Chirurgiegerät (1) nach Anspruch 1, **dadurch gekennzeichnet, dass** der Buchsenkontakt (3) so angeordnet ist, dass er den Steckkontakt (12) in eingestecktem Zustand am distalen Ende des Steckkontakts kontaktiert.

3. Hochfrequenz-Chirurgiegerät (1) nach einem der oben genannten Ansprüche, **dadurch gekennzeichnet, dass** der Buchsenkontakt (3) so angeordnet ist, dass er den Steckkontakt (12) beim Einschieben erst kontaktiert nachdem die Raste im Wesentlichen vollständig in die Gegenraste eingerastet ist.

4. Hochfrequenz-Chirurgiegerät (1) nach einem der oben genannten Ansprüche, **dadurch gekennzeichnet, dass** der Buchsenkontakt (3) aus einem federnden Material hergestellt und vom Steckkontakt (12) im eingesteckten Zustand federnd auslenkbar angeordnet ist.

5. Hochfrequenz-Chirurgiegerät (1) nach Anspruch 3, **dadurch gekennzeichnet, dass** die Raste aus einem federnden Material hergestellt ist, wobei die Federkonstante der Raste größer ist als die des Buchsenkontakts.(3)

6. Hochfrequenz-Chirurgiegerät (1) nach einem der oben genannten Ansprüche, **dadurch gekennzeichnet, dass** der Buchsenkontakt (3) im Wesentlichen gabelförmig oder geschlitzt ausgestaltet ist und so angeordnet ist, dass die Achse des rotationssymmetrischen Steckkontakts (12) vom Kontakt ausgespart ist.

7. Hochfrequenz-Chirurgiegerät (1) nach einem der oben genannten Ansprüche, **dadurch gekennzeichnet, dass** die Ausgangsbuchse (2) für die Aufnahme eines einpoligen Rundsteckers, insbesondere eines Bovie Steckers oder Olympus 6mm Rundsteckers, ausgebildet ist.

## Claims

1. High-frequency surgical device (1) for producing high-frequency energy for the separation and/or coagulation of biological tissue, having at least one energy source (21) and having at least one output socket (2) into which an elongate electrically conductive plug type contact (12) of an electrosurgical instrument (6) can be inserted and which comprises a catch (17) for engagement in a counter-catch (13) which is formed in the plug type contact, and a socket contact (3) which is electrically isolated from the catch (17) and which is electrically connected to the energy source (21),
**characterised in that**
the greatest possible spacing of a contact point with the plug type contact (12) with respect to the outer side of the high-frequency surgical device (1) is greater with the socket contact (3) than with the catch (17).

2. High-frequency surgical device (1) according to claim 1, **characterised in that** the socket contact (3) is arranged in such a manner that it contacts the plug type contact (12) in the inserted state at the distal end of the plug type contact.

3. High-frequency surgical device (1) according to either of the preceding claims, **characterised in that** the socket contact (3) is arranged in such a manner that it contacts the plug type contact (12) when being inserted only after the catch is substantially completely engaged in the counter-catch.

4. High-frequency surgical device (1) according to any one of the preceding claims, **characterised in that** the socket contact (3) is produced from a resilient material and is arranged so as to be able to be resiliently deflected by the plug type contact (12) in the inserted state.

5. High-frequency surgical device (1) according to claim 3, **characterised in that** the catch is produced from a resilient material, the spring constant of the catch being greater than that of the socket contact (3).

6. High-frequency surgical device (1) according to any one of the preceding claims, **characterised in that** the socket contact (3) is constructed in a substantially fork-like or slotted manner and is arranged in such a manner that the axis of the rotationally symmetrical plug type contact (12) is spaced-apart from the contact.

7. High-frequency surgical device (1) according to any one of the preceding claims, **characterised in that** the output socket (2) is constructed for receiving a single-pole round plug, in particular a Bovie plug or Olympus 6mm round plug.

## Revendications

1. Appareil chirurgical à haute fréquence (1) destiné à générer de l'énergie à haute fréquence en vue de la séparation et/ou coagulation d'un tissu biologique, ledit appareil comportant au moins une source d'énergie (21) et au moins une prise femelle de sortie (2) dans laquelle peut être enfiché un contact mâle (12) allongé et électriquement conducteur d'un instrument électro-chirurgical (6) et qui comporte un élément d'encliquetage (17) destiné à s'encliqueter dans un élément d'encliquetage correspondant (13) formé dans le contact mâle, et un contact femelle (3) qui est électriquement isolé de l'élément d'encliquetage (17) et qui est relié électriquement à la source d'énergie (21), **caractérisé en ce que** la plus grande distance possible d'un point de contact avec le contact mâle (12) au côté extérieur de l'appareil chirurgical à haute fréquence (1) est plus grande au niveau du contact femelle (3) qu'au niveau de l'élément d'encliquetage (17).

2. Appareil chirurgical à haute fréquence (1) selon la revendication 1, **caractérisé en ce que** le contact femelle (3) est disposé de telle sorte qu'il vient en contact avec le contact mâle (12) lorsque celui-ci est enfiché à l'extrémité distale du contact mâle.

3. Appareil chirurgical à haute fréquence (1) selon l'une des revendications susmentionnées, **caractérisé en ce que** le contact femelle (3) est disposé de telle sorte qu'il ne vient en contact avec le contact mâle (12) lors de l'insertion qu'après que l'élément d'encliquetage est sensiblement totalement inséré dans l'élément d'encliquetage correspondant.

4. Appareil chirurgical à haute fréquence (1) selon l'une des revendications susmentionnées, **caractérisé en ce que** le contact femelle (3) est fabriqué à partir d'un matériau élastique et est disposé de façon à pouvoir être dévié élastiquement par le contact mâle (12) lorsque celui-ci est enfiché.

5. Appareil chirurgical à haute fréquence (1) selon la revendication 3, **caractérisé en ce que** l'élément d'encliquetage est fabriqué à partir d'un matériau élastique, la constante d'élasticité de l'élément d'encliquetage étant supérieure à celle du contact femelle (3).

6. Appareil chirurgical à haute fréquence (1) selon l'une des revendications susmentionnées, **caractérisé en ce que** le contact femelle (3) a une conformation sensiblement fourchue ou fendue et est disposée de telle sorte que l'axe du contact mâle (12) à symétrie de révolution est dégagé du contact.

7. Appareil chirurgical à haute fréquence (1) selon l'une des revendications susmentionnées, **caractérisé en ce que** la prise femelle de sortie (2) est conformée pour recevoir un connecteur coaxial unipolaire, notamment un connecteur Bovie ou un connecteur coaxial Olympus 6 mm.
